## Europäisches Patentamt
## European Patent Office
## Office européen des brevets

(19)

(11) Veröffentlichungsnummer : **0 061 057**
**B1**

(12)

# EUROPÄISCHE PATENTSCHRIFT

(45) Veröffentlichungstag der Patentschrift :
**15.08.84**

(21) Anmeldenummer : **82101813.2**

(22) Anmeldetag : **08.03.82**

(51) Int. Cl.³ : **C 07 C 29/74, C 07 C 31/04//**
**C07C45/38, C07C47/052**

---

(54) **Verfahren zur Herstellung von Formaldehyd.**

---

(30) Priorität : **19.03.81 DE 3110723**

(43) Veröffentlichungstag der Anmeldung :
**29.09.82 Patentblatt 82/39**

(45) Bekanntmachung des Hinweises auf die Patenterteilung : **15.08.84 Patentblatt 84/33**

(84) Benannte Vertragsstaaten :
**AT BE DE FR GB IT NL**

(56) Entgegenhaltungen :
**DD-A-  140 175**
**DE-B- 1 101 383**
**DE-B- 1 618 719**

(73) Patentinhaber : **BAYER AG**
**Konzernverwaltung RP Patentabteilung**
**D-5090 Leverkusen 1 Bayerwerk (DE)**

(72) Erfinder : **Klinkmann, Kurt**
**Stephan-Lochner-Strasse 35**
**D-5090 Leverkusen 17 (DE)**
Erfinder : **Wambach, Raimund, Dr.**
**Berta-von-Suttner-Strasse 65**
**D-5090 Leverkusen 1 (DE)**

**Beschreibung**

Die Erfindung betrifft ein Verfahren zur Herstellung von Formaldehyd durch Oxidation von Methanol oder eines Methanol/Wasser-Gemisches am Silberkontakt in der Gasphase.

Es ist bekannt, daß die zur Formaldehyd-Herstellung benötigten Ausgangsmaterialien sehr rein sein müssen, um die Aktivität und Lebensdauer des Silberkatalysators nicht allzu stark zu beeinträchtigen (vgl. z. B. DE-AS 2 323 758). Das zur Umsetzung eingesetzte Methanol oder das Methanol/Wasser-Gemisch enthält praktisch immer neben einer Anzahl organischer Substanzen noch eine Reihe anorganischer Verbindungen, die beispielsweise ausbeutevermindernde Nebenreaktionen begünstigen oder als Katalysatorgifte wirken. Es ist daher erforderlich, das Methanol vor der Oxidation zu reinigen und zur Herstellung des Methanol/Wasser-Gemisches sehr reines Wasser zu verwenden oder das Methanol/Wasser-Gemisch nachzureinigen.

So ist aus der DE-AS 1 235 881 ein Verfahren zur Herstellung von Formaldehyd bekannt, bei dem man das Rohmethanol vor, während und/oder nach der Abnahme einer niedriger als Methanol siedenden Fraktion und vor der Umsetzung zu Formaldehyd mit Alkali behandelt. Eine ähnliche Reinigungsmethode ist auch aus der britischen Patentschrift 344 796 bekannt. Nach dieser Patentschrift wird z. B. Rohmethanol, das als Verunreinigung Spuren von Eisen enthält, ebenfalls mit Alkalien behandelt. Die Reinigung mit Alkalien hat jedoch den Nachteil, daß große Mengen stark alkalischer Abwässer anfallen, die mit einem hohen technischen Aufwand beseitigt werden müssen, was zu Lasten der Wirtschaftlichkeit solcher Reinigungsverfahren geht.

Eine andere Möglichkeit Rohmethanol zu reinigen besteht in der Behandlung mit Oxidationsmitteln. Beispielsweise ist aus der britischen Patentschrift 500 745 eine Reinigung des Rohmethanols durch Behandlung mit Chromsäure, aus der DE-PS 625 324 eine Reinigung mit Permanganat und Zinkchlorid und aus der DE-AS 1 152 392 eine Reinigung durch Behandlung mit ozonhaltigem Gas bekannt. Nach der DE-AS 1 231 677 soll es besonders wirkungsvoll sein, Rohmethanol durch Behandlung mit Sauerstoff oder Wasserstoffperoxid in Gegenwart von großflächigen Stoffen, wie Kieselgel, Kieselgur, Tonerde, Bleierde oder Aktivkohle zu reinigen.

Die genannten Reinigungsverfahren haben jedoch den Nachteil, daß bei der Verwendung von Chromsäure oder Permanganat Abwasserprobleme auftreten. Außerdem können Spuren von Chromsäure und Permanganat, die im behandelten Methanol verbleiben, die Aktivität des empfindlichen Silberkatalysators beeinträchtigen. Auch der Einsatz von Ozon oder Wasserstoffperoxid bei der Behandlung des Rohmethanols bringt keinerlei Vorteile, da zum einen diese Verbindungen relativ teure Chemikalien darstellen, die nach der Behandlung nicht wiedergewonnen werden können, und zum anderen damit nur oxidierbare Substanzen entfernt werden können.

Wie der DE-AS 2 323 758, Spalte 4, Zeilen 14-29 zu entnehmen ist, sind trotz der Reiningung des Rohmethanols mit den zuvor erwähnten Verbindungen die Lebensdauer des Silberkatalysators sowie die mit ihm erzielten Ausbeuten und Raum/Zeit-Ausbeuten an reinem Formaldehyd noch unbefriedigend, da ein Teil der im Rohmethanol enthaltenen Verunreinigungen nicht abgetrennt werden und während der Reaktion den Katalysator inaktivieren.

Weiterhin sind aus der DE-AS 1 101 383, der DE-AS 1 618 719 und der DD-PS 146 175 Verfahren zur Methanol Reinigung durch Behandlung des Methanols mit Ionenaustauschern bekannt.

Es wurde nun ein Verfahren zur Herstellung von Formaldehyd durch Oxidation von Methanol oder eines Methanol/Wasser-Gemisches am Silberkontakt in der Gasphase gefunden, das dadurch gekennzeichnet ist, daß man das zu oxidierende Methanol oder das Methanol/Wasser-Gemisch vor der Verdampfung mit einem sauren und gegebenenfalls einem basischen Ionenaustauscher behandelt und den Verdampfersumpf über einen sauren Ionenaustauscher umpumpt.

Als saure Ionenaustauscher können in das erfindungsgemäße Verfahren die handelsüblichen sauren, makroporösen oder gelförmigen Kationenaustauscher in der $H^\oplus$-Form auf der Basis von sulfonsäuregruppenhaltigen, vernetzten Polystyrol-harzen (stark saure Ionenaustauscher) oder auf der Basis von carboxylgruppenhaltigen, vernetzten Polyacrylsäuren (schwach saure Ionenaustauscher) eingesetzt werden, wie sie z. B. in Ullmanns Encyklopädie der technischen Chemie, 4. Auflage, Band 13, Seite 279-346, beschrieben sind.

Bevorzugt werden in das erfindungsgemäße Verfahren stark saure, makroporöse Kationenaustauscher eingesetzt.

Als basische Ionenaustauscher können die handelsüblichen basischen makroporösen oder gelförmigen Anionenaustauscher auf der Basis von quarternären Ammoniumgruppenhaltigen, vernetzten Polystyrol- oder Polyacrylamidharzen (stark basisch) oder auf Basis von Aminogruppenhaltigen, vernetzten Polystyrol- oder Polyacrylamidharzen (schwach basisch) eingesetzt werden, wie sie z. B. in Ullmanns Encyklopädie der technischen Chemie, 4. Auflage, Band 13, Seite 279-346, beschrieben sind.

Bevorzugt werden in das erfindungsgemäße Verfahren stark basische, makroporöse Anionenaustauscher eingesetzt.

Für die Behandlung des Methanols oder des Methanol-Wassergemisches setzt man üblicherweise etwa 100 bis 200 l, bevorzugt 150 bis 180 l, saure Ionenaustauscher in das erfindungsgemäße Verfahren ein.

Bei diesen Mengen an eingesetzten sauren Ionenaustauschern können etwa 500 bis 10 000 l/h,

2

# 0 061 057

bevorzugt 2 000 bis 5 000 l/h, wasserfreies oder wäßriges Methanol durchgesetzt werden. Selbstverständlich können auch kleinere oder größere Mengen an sauren Ionenaustauschern eingesetzt werden, wobei sich dann die durch zusetzende Menge an wasserfreiem oder wäßrigem Methanol entsprechend verringert oder erhöht.

Die Behandlung des Methanols oder des Methanol/Wassergemisches mit den sauren Ionenaustauschern wird im allgemeinen bei Temperaturen von etwa 0 bis 60 °C, bevorzugt bei 15 bis 40 °C, durchgeführt, wobei vorteilhafterweise bei Normaldruck gearbeitet, wird.

Wird das umzusetzende Methanol oder das Methanol/Wassergemisch noch zusätzlich mit basischen Ionenaustauschern behandelt, so setzt man ebenfalls etwa 100 bis 200 l, bevorzugt 150 bis 180 l, an basischen Ionenaustauschern ein. Auch hier können, falls dies zweckmäßig erscheint, sowohl kleinere als auch größere Mengen an basischen Ionenaustauschern eingesetzt werden, wobei sich selbstverständlich die Durchsatzmenge an wasserfreiem oder wäßrigem Methanol entsprechend verringert oder vergrößert. Bei der oben angegebenen Menge an basischen Ionenaustauschern kann man etwa 300 bis 3 000 l/h, bevorzugt 400 bis 800 l/h, wasserfreies oder wäßriges Methanol durchsetzen.

Üblicherweise wird die Behandlung mit basischen Ionenaustauschern bei Temperaturen von etwa 0 bis 70 °C, bevorzugt bei 20 bis 65 °C, bei Normaldruck durchgeführt.

Es ist jedoch auch möglich, bei erhöhtem Druck von etwa 0,1 bis 5, bevorzugt 2 bis 3, bar zu arbeiten, wobei Temperaturen von etwa 90 bis 100 °C am günstigsten sind. Die Anwendung von Druck ist vorteilhaft, da dadurch die Durchsatzmengen an Methanol oder an dem Methanol/Wassergemisch beträchtlich erhöht werden können, ohne daß dabei die Reinigungswirkung beeinträchtigt würde.

Wird bei der Umsetzung wäßriges Methanol eingesetzt, so beträgt dessen Konzentration etwa 50 bis 90, bevorzugt 55 bis 70, Gew.-%.

Die Behandlung des Methanols oder des Methanol/Wassergemisches mit den sauren und gegebenenfalls den basischen Ionenaustauschern kann in den üblichen Ionenaustauschern kann in den üblichen Ionenaustauscherapparaten und nach üblichen Behandlungsmethoden erfolgen. Dabei kann man zunächst das Methanol oder das Methanol/Wassergemisch mit saurem Ionenaustauscher behandeln und anschließend, falls erforderlich, mit basischem Ionenaustauscher. Möglich ist natürlich auch die umgekehrte Behandlungsweise. Aus praktischen Gründen ist die Behandlung zuerst mit alkalischen Ionenaustauschern und dann mit sauren Ionenaustauschern bevorzugt. Weiterhin ist es möglich, die Behandlung des Methanols oder des Methanol/Wassergemisches in einer Mischbett- oder einer Schichtbettapparatur vorzunehmen.

Die erfindungsgemäße Behandlung des Methanols oder des Methanol/Wassergemisches kann sowohl kontinuierlich als auch diskontinuierlich durchgeführt werden.

Beispielsweise kann man die Behandlung des wäßrigen Methanols (Konzentration an Methanol ca. 56 Gew.-%) so durchführen, daß man das Methanol bei 40-100 °C, bevorzugt bei 50-60 °C, und bei einem Druck von bis zu ca. 10 bar in einer Menge von 4 000 l/h durch einen mit 1 200 l stark basischen Ionenaustauscher gefüllten Behälter pumpt und anschließend das so behandelte wäßrige Methanol bei Raumtemperatur und bei einem Druck von bis zu 10 bar durch einen mit 200 l stark sauren Ionenaustauscher gefüllten Behälter in einer Menge von 4 000 l/h leitet.

Das dabei erhaltene wäßrige Methanol kann dann direkt ohne weitere Reinigung verdampft und am Silberkontakt zu Formaldehyd umgesetzt werden.

Gegenüber den bislang aus dem Stand der Technik bekannten Verfahren zur Herstellung von Formaldehyd aus Methanol, die das umzusetzende Methanol z. B. durch Destillation, Behandlung mit Alkalien oder Oxidationsmitteln reinigen, wird durch das erfindungsgemäße Verfahren eine wesentlich erhöhte Katalysatorlebensdauer (Steigerung von 120 Tagen gemäß DE-OS 2 137 938 auf mindestens 166 Tage nach dem erfindungsgemäßen Verfahren, wobei zu bemerken ist, daß der Versuch wegen einer Technischen Störung unterbrochen werden mußte, obwohl der Katalysator noch voll aktiv war) und eine höhere Ausbeute an Formaldehyd erreicht. Außerdem gelingt es durch die erfindungsgemäße Behandlung des Methanols oder des Methanol/Wassergemisches, den Restmethanolgehalt im Formaldehyd unter 1 Gew.-% (gaschromatografisch bestimmt) zu senken. Da nur noch geringe Mengen an Methanol im Formaldehyd verbleiben, kann der Formaldehyd in praktisch allen Fällen direkt weiter umgesetzt werden, ohne daß sich das Methanol störend bemerkbar machen würde.

Durch die mit Hilfe des erfindungsgemäßen Verfahrens erzielten Ausbeuteverbesserungen an Formaldehyd und durch die erreichten längeren Katalysatorstandzeiten erhöht sich die Kapazität einer vorhandenen Anlage um etwa 10 %, was einen zusätzlichen wirtschaftlichen Vorteil darstellt.

Obwohl man bei dem erfindungsgemäßen Verfahren die Behandlung des Methanols mit Ionenaustauschern durchführt, die aus organischen Polymeren aufgebaut sind, und man dabei annehmen mußte, daß sich Spuren des Ionenaustauschermaterials im Methanol lösen und damit die Aktivität des empfindlichen Silberkatalysators beeinträchtigen würden, ist es für den Fachmann außerordentlich überraschend, daß sich entgegen der Erwartung die Lebensdauer des Silberkatalysators beträchtlich erhöhte und die Formaldehyd-Ausbeute verbesserte.

Das nachstehend aufgeführte Beispiel soll das erfindungsgemäße Verfahren verdeutlichen.

## Beispiel

Das für die Formaldehyd-Herstellung bestimmte wäßrige, destillierte Methanol (ca. 56 % Gew.-%

3

Methanolgehalt) wurde bei einer Temperatur von 15 bis 30 °C mit einem stark sauren Kationenaustauscher in der H⊕-Form auf der Basis von sulfonsäuregruppenhaltigem Polystyroldivinylbenzolharz (im Handel z. B. als Lewatit® SC 108, S 100, SP 112 und SP 120 erhältlich ; Füllmenge : 300 l ; Durchsatz : 4 000 l/h) kontinuierlich behandelt und dann direkt in den Verdampfer einer technischen Formaldehydanlage geleitet. Zusätzlich wurde der Verdampfersumpf kontinuierlich in einer Menge von 1 000 l/h über einem mit 120 l stark sauren Kationenaustauscher (H⊕-Form) gefüllten Behälter umgepumpt. Das im Verdampfer verdampfte Methanol/Wassergemisch, dem noch Luft zugemischt wurde, wurde über einen Silberkontakt geblasen, der dem in der DE-PS 1 285 995, Beispiel 1, beschriebenen Silberkatalysator entsprach.

Die Umsetzung am Katalysator wurde mit einem Gemisch bestehend aus :

2 060 kg/h Methanoldampf
1 586 kg/h Wasserdampf und
4 020 kg/h Luft

bei einem Druck von etwa 150 bis 300 mbar und bei einer Temperatur von 660 bis 670 °C durchgeführt.

Die Reaktionsgase — Formaldehyd, Wasser und Restmethanol — wurden nach Abkühlen im Reaktor-Kühler in einer gebraüchlichen Absorptionsanlage kondensiert und das Abgas zusätzlich mit Wasser gewaschen, das wieder zur Formaldehydabsorption verwendet wurde.

Während einer Fahrperiode von 166 Tagen wurde kontinuierlich eine ca. 37,5 gew.-%ige Formaldehydlösung mit einem Restmethanolgehalt von 0,6 bis 0,9 Gew.-% erhalten. Wie sich der Restmethanolgehalt in der Formaldehydlösung während dieser Zeit änderte, zeigt die nachfolgende Tabelle :

|  | | Restmethanolgehalt (gaschromatographisch bestimmt) in Gew.-% | Formaldehyd-Konzentration in Gew.-% |
|---|---|---|---|
| 1 | Tag | 0,6 | 37,3 |
| 10 | Tage | 0,7 | 37,5 |
| 50 | Tage | 0,6 | 37,1 |
| 100 | Tage | 0,7 | 37,8 |
| 120 | Tage | 0,8 | 37,3 |
| 140 | Tage | 0,9 | 37,5 |
| 150 | Tage | 0,9 | 37,3 |
| 160 | Tage | 0,9 | 37,4 |
| 166 | Tage | 0,9 | 37,2 |

Die Formaldehydausbeute schwankte während dieser Fahrperiode zwischen 89 und 91 % der Theorie.

Wurde umzusetzendes wäßriges Methanol, das destillativ gereinigt worden war aber noch Spuren an Eisencarbonyl enthielt (ca. 1,5 mg Fe/l), zusätzlich noch mit einem stark basischen Ionenaustauscher behandelt, um die Eisencarbonylverbindungen aus dem Methanol zu entfernen, so wurde eine Formaldehydlösung erhalten, deren Restmethanolgehalt ebenfalls bei 0,6 bis 0,9 Gew.-% lag. Die Formaldehydausbeute schwankte ebenfalls zwischen 89 und 91 % der Theorie.

Nach 166 Tagen mußte der Versuch wegen einer technischen Störung unterbrochen werden. Zu diesem Zeitpunkt war der Katalysator noch voll wirksam. Der Katalysator war praktisch frei von störenden metallischen oder kohlehaltigen Ablagerungen.

Vergleichsbeispiel

Das für die Formaldehyd-Herstellung bestimmte wäßrige, destillierte Methanol (ca. 56 Gew.-% Methanolgehalt) wurde bei Raumtemperatur direkt in den Verdampfer einer technischen Formaldehydanlage geleitet, wobei gleichzeitig Luft in den Verdampfer eingeblasen wurde. Der Silberkatalysator entsprach dem in der DE-PS 1 285 995, Beispiel 1, beschriebenen Katalysator. Die Umsetzung am Katalysator wurde mit einem Gemisch bestehend aus :

2 060 kg/h Methanoldampf
1 586 kg/h Wasserdampf und
4 020 kg/h Luft

· bei einem Druck von etwa 100 bis 300 mbar und einer Temperatur von etwa 650 bis 680 °C durchgeführt.

Die Reaktionsgase — Formaldehyd, Wasser und Restmethanol — wurden nach Abkühlen im Reaktor-Kühler in einer gebräuchlichen Absorptionsanlage kondensiert und das Abgas zusätzlich mit Wasser gewaschen, das wieder bei der Formaldehydabsorption verwendet wurde.

Während einer Fahrperiode von 80 Tagen wurde kontinuierlich eine ca. 37,5 gew.-%ige Formaldehydlösung mit Restmethanolgehalten erhalten, die zwischen 1,0 und 1,8 Gew.-% schwankt. Wie sich der Restmethanolgehalt im Formaldehyd änderte, zeigt die nachfolgende Tabelle :

|  |  | Restmethanolgehalt (gaschromatographische Bestimmung) in Gew.-% | Formaldehyd-Konzentration in Gew.-% |
|---|---|---|---|
| 1 | Tag | 1,0 | 37,3 |
| 3 | Tage | 1,05 | 37,6 |
| 5 | Tage | 1,25 | 37,3 |
| 7 | Tage | 1,7 | 37,8 |
| 10 | Tage | 1,35 | 37,2 |
| 20 | Tage | 1,4 | 37,5 |
| 40 | Tage | 1,6 | 37,4 |
| 60 | Tage | 1,75 | 37,3 |
| 80 | Tage | 1,8 | 37,5 |

Die Formaldehydausbeute lag bei dieser Fahrperiode bei etwa 86 bis 89 % der Theorie.

Die Aktivität des Katalysators ließ, wie der ansteigende Restmethanolgehalt der hergestellten Formaldehydlösung zeigt, mit zunehmender Laufzeit nach.

Der Katalysator enthielt eine Reihe von Fremdmetallen und war zusätzlich durch Kohleablagerungen verunreinigt.

**Ansprüche**

1. Verfahren zur Herstellung von Formaldehyd durch Oxidation von Methanol oder eines Methanol/Wassergemisches am Silberkontakt in der Gasphase, dadurch gekennzeichnet, daß man das zu oxidierende Methanol oder das Methanol/Wassergemisch vor der Verdampfung mit einem sauren und gegebenenfalls einem basischen Ionenaustauscher behandelt und den Verdampfersumpf über einen sauren Ionenaustauscher umpumpt.

2. Verfahren nach Anspruch 1, dadurch gekennzeichnet, daß man saure, makroporöse oder gelförmige Kationenaustauscher in der $H^{\oplus}$-Form auf der Basis von Sulfonsäuregruppen-haltigen, vernetzten Polystyrol-harzen oder auf Basis von Carboxylgruppen-haltigen, vernetzten Polyacrylsäuren einsetzt.

3. Verfahren nach Anspruch 1, dadurch gekennzeichnet, daß man basische, makroporöse oder gelförmige Anionenaustauscher auf der Basis von quarternären Ammoniumgruppen-haltigen, vernetzten Polystyrol- oder Polyacrylamidharzen oder auf Basis von Aminogruppen-haltigen, vernetzten Polystyrol-oder Polyacrylamidharzen einsetzt.

4. Verfahren nach Ansprüchen 1 und 2, dadurch gekennzeichnet, daß man die Behandlung mit sauren Ionenaustauschern bei Temperaturen von 0 bis 60 °C durchführt.

5. Verfahren nach Ansprüchen 1 und 3, dadurch gekennzeichnet, daß man die Behandlung mit basischen Ionenaustauschern bei Temperaturen von 0 bis 70 °C durchführt.

6. Verfahren nach Ansprüchen 1 und 3, dadurch gekennzeichnet, daß man die Behandlung mit basischen Ionenaustauschern bei einem Druck von 0,1 bis 5 bar und bei Temperaturen von 90 bis 100 °C durchführt.

**Claims**

1. Process for the preparation of formaldehyde by the oxidation of methanol or of a methanol/water mixture in the gas phase in the presence of a silver catalyst, characterised in that the methanol to be oxidised or the methanol/water mixture is treated, before vaporisation, with an acid ion exchanger and, if appropriate, with a basic ion exchanger and the vaporiser sump product is passed through an acid ion exchanger.

2. Process according to Claim 1, characterised in that acid, macroporous or gel-type cation exchangers in the $H^{\oplus}$ form, based on crosslinked polystyrene resins containing sulphonic acid groups or based on crosslinked polyacrylic acids containing carboxyl groups, are employed.

3. Process according to Claim 1, characterised in that basic, macroporous or gel-type anion exchangers based on crosslinked polystyrene or polyacrylamide resins containing quaternary ammonium groups, or based on crosslinked polystyrene or polyacrylamide resins containing amino groups, are employed.

4. Process according to Claims 1 and 2, characterised in that the treatment with acid ion exchangers is carried out at temperatures of 0 to 60 °C.

5. Process according to Claims 1 and 3, characterised in that the treatment with basic ion exchangers is carried out at temperatures of 0 to 70 °C.

6. Process according to Claims 1 and 3, characterised in that the treatment with basic ion exchangers is carried out under a pressure of 0.1 to 5 bars and at temperatures of 90 to 100 °C.

**Revendications**

1. Procédé de fabrication de formaldéhyde par oxydation du méthanol ou d'un mélange de méthanol/eau sur un catalyseur d'argent en phase gazeuse, caractérisé en ce qu'on traite le méthanol ou le mélange de méthanol/eau à oxyder, avant l'évaporation, avec un échangeur d'ions acide et éventuellement un échangeur d'ions basique et l'on recycle par pompage le produit de queue de l'évaporateur avec passage sur un échangeur d'ions acide.

2. Procédé selon la revendication 1, caractérisé en ce qu'on utilise des échangeurs de cations acides, macroporeux ou sous forme de gels en la forme $H^{\oplus}$, à base de résines de polystyrène réticulées renfermant des groupes acide sulfonique, ou à base d'acides polyacryliques réticulés renfermant des groupes carboxyle.

3. Procédé selon la revendication 1, caractérisé en ce qu'on utilise des échangeurs d'anions basiques, macroporeux ou sous forme de gels, à base de résines de polystyrène ou de polyacrylamide réticulées renfermant des groupes d'ammonium quaternaires, ou à base de résines de polystyrène ou de polyacrylamide réticulées renfermant des groupes amino.

4. Procédé selon les revendications 1 et 2, caractérisé en ce qu'on exécute le traitement avec des échangeurs d'ions acides à des températures de 0 à 60 °C.

5. Procédé selon les revendications 1 et 3, caractérisé en ce qu'on exécute le traitement avec des échangeurs d'ions basiques à des températures de 0 à 70 °C.

6. Procédé selon les revendications 1 et 3, caractérisé en ce qu'on exécute le traitement avec des échangeurs d'ions basiques sous une pression de 0,1 à 5 bars et à des températures de 90 à 100 °C.